Europäisches Patentamt

**(19)** European Patent Office

Office européen des brevets

**(11)** Numéro de publication: **0 245 332**
**B1**

**(12)** FASCICULE DE BREVET EUROPEEN

**(45)** Date de publication du fascicule du brevet: **24.10.90**

**(51)** Int. Cl.⁵: **A 61 B 17/425,** A 61 B 19/02

**(21)** Numéro de dépôt: **86906357.8**

**(22)** Date de dépôt: **07.11.86**

**(86)** Numéro de dépôt international:
**PCT/FR86/00378**

**(87)** Numéro de publication internationale:
**WO 87/02879 21.05.87 Gazette 87/11**

**(54)** CONTENEUR POUR FECONDATION DES OVOCYTES HUMAINS EN ABSENCE D'AIR ENRICHI EN CO 2?.

**(30)** Priorité: **08.11.85 FR 8516558**

**(43)** Date de publication de la demande:
**19.11.87 Bulletin 87/47**

**(45)** Mention de la délivrance du brevet:
**24.10.90 Bulletin 90/43**

**(84)** Etats contractants désignés:
**AT BE CH DE FR GB IT LI LU NL SE**

**(56)** Documents cités:
**EP-A-0 131 166**
**FR-A-2 194 453**
**US-A-2 456 607**
**US-A-4 380 997**

**(73)** Titulaire: **RANOUX, Claude**
**7, rue des Grands-Champs**
**F-77330 Lésigny (FR)**

**(72)** Inventeur: **RANOUX, Claude**
**7, rue des Grands-Champs**
**F-77330 Lésigny (FR)**

**(74)** Mandataire: **CABINET BONNET-THIRION**
**95 Boulevard Beaumarchais**
**F-75003 Paris (FR)**

Courier Press, Leamington Spa, England.

**Description**

Cette invention concerne un procédé de fécondation d'ovocytes et de culture des embryons humains ainsi obtenu, ainsi qu'un dispositif de mise en oeuvre de ce procédé.

On connaît déjà du brevet US 4 380 997 (LEIBO) un procédé et un conteneur de conservation d'un embryon à la température de l'azote liquide. Le conteneur en forme de tube contient un milieu liquide immergant un embryon, ledit tube étant pourvu d'un orifice adapté à être fermé de manière étanché par un bouchon.

Le milieu liquide d'immersion de l'embryon est une solution cryoprotectrice permettant de le congeler sans dommages à la température de l'azote liquide et de le conserver à l'état congelé pendant plusieurs années. Cependant lorsque l'embryon est décongelé il convient de diluer l'agent cryoprotecteur du milieu d'immersion de l'embryon si l'on veut que celui-ci reste viable car la solution cryoprotectrice a une toxicité vis-à-vis de l'embryon, à la température ordinaire. La solution cryoprotectrice est donc diluée par exemple par une solution de sucrose ayant la même osmolarité que celle de la solution cryoprotectrice.

Le but fixé par la présente demande est de trouver un procédé et un dispositif simples permettant la fécondation in-vitro d'un ovocyte humain par des spermatozoïdes suivie du clivage en embryons de l'ovocyte fécondé.

La fécondation in-vitro des ovocytes humains est une technique très complexe qui a permis de solutionner des cas de stérilité du couple jusqu'alors irréversibles. Depuis la première naissance, en 1978, de Louise Brown, réalisée par l'équipe d'Edwards en Angleterre, des milliers d'enfants sont nés par cette technique à travers le monde.

Le souci majeur de l'ensemble des équipes de fécondation in-vitro a toujours été d'obtenir une simplification de la technique tout en conservant ou en améliorant les résultats. C'est ainsi que sur le plan clinique sont apparatus:

la stimulation, afin d'obtenir plusieurs ovocytes et d'augmenter ainsi les chances de succès,

d'autres modes de ponction que celui sous contrôle coelioscopique, notamment ceux effectués sous contrôle échographique transvésical puis transvaginal et enfin transurétral, qui permettent de se passer de l'anesthésie générale et d'éviter la toxicité éventuelle du $CO_2$. Sur le plan biologique la congélation des embryons surnuméraires a permis une amélioration des résultats. Seule l'étape biologique de la fécondation proprement dite n'a subi que de très minimes modifications.

Cette étape biologique demeurait jusqu'à ce jour très complexe. Classiquement elle correspondait à une culture des embryons aérobie et stérile en boîte ou en tube à 37° et sous 5% de $CO_2$. Ce qui nécessite des boites et tubes non hermétiques, avec un risque de contamination par le milieu ambiant. D'où la nécessité de disposer d'une étuve à $CO_2$ ou d'une couveuse (mise au point par "Testart"), parfaitement équilibrée à 37° et en $CO_2$. Celles-ci sont des matériels lourds et chers.

De même initialement après la ponction, on procédait à une phase de maturation de 1 à 4 heures des ovocytes dans le milieu de culture complémenté le plus souvent en sérum humain et après ce délai à la fécondation des ovocytes après changement de milieu; 18 à 24 heures après, une dénudation des oeufs était effectuée. (Cette dénudation correspond à retirer de façon mécanique les cellules du cumulus entourant l'oeuf afin d'apprécier son stade de développement). L'oeuf était alors transféré dans un nouveau milieu, qui, 20 à 24 heures après était à nouveau changé avant le replacement dans la cavité utérine, ce qui correspondait à l'utilisation de plus de 3 ml de milieu de culture par oeuf et à de nombreuses manipulations échelonnées sur 48 heures, pouvant créer une toxicité pour ces oeufs.

Le procédé selon l'invention est énoncé dans la revendication 1. Ce procédé est simple et économique aussi bien en temps que financièrement.

Ce procédé consiste en une fécondation en absence d'air enrichi en $CO_2$ des ovocytes humains à l'aide d'un tube étanche entièrement rempli de milieu de culture et de gamètes et placé dans la cavité vaginale qui joue alors rôle d'étuve. Dès la ponction des ovocytes, dont l'heure a été retardée (afin de se passer de la phase de maturation ovocytaire), ceux-ci sont placés dans le conteneur ainsi que les spermatozoïdes nécessaires à la fécondation et qui ont été préalablement préparés. Puis le dispositif, avec son mode de contention sont placés dans la cavité vaginale où ils ne seront reitrés qu'après 44 à 48 heures afin de replacer immédiatement les oeufs dans la cavité utérine à l'aide d'un cathéter de "Frydman".

Le dispositif de mise en oeuvre du procédé est obtenu selon l'énoncé de la revendication 8. L'invention sera mieux comprise à l'aide de la description qui va suivre en référence aux dessins annexés où

la figure 1 montre une partie du dispositif de mise en oeuvre de l'invention;

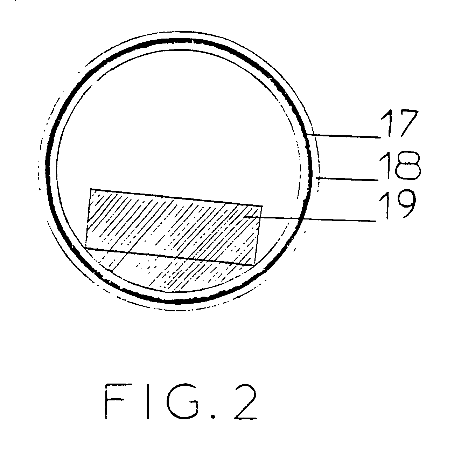

la figure 2 montre un moyen de contention faisant partie de ce dispositif.

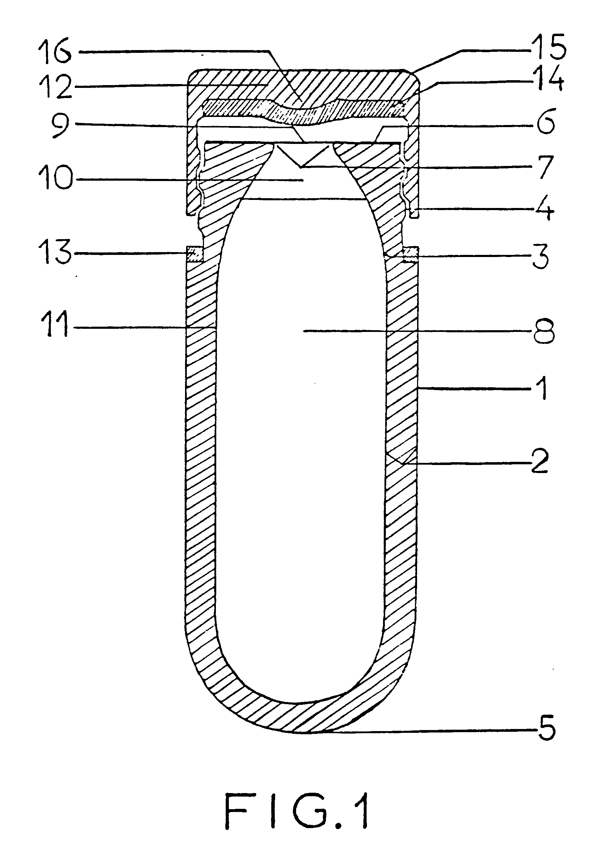

Nous allons vous décrire un modèle du conteneur qui constitue le dispositif (Fig. 1).

C'est un tube cylindrique de faibles dimensions, aux parois externes lisses et arrondies (1) afin d'éviter tout traumatisme de la muqueuse vaginale. Ses dimensions sont d'environ 4 cm de long pour un diamètre externe de 1,5 cm; l'épaisseur des parois est de 2 mm sur le corps du cylindre (2) sauf au niveau du col où elle n'est plus que de 1 mm (3), ce qui donne une cavité interne de 1,1 cm de diamètre sur 3,8 cm de long avec un volume utile d'environ 3,2 cm3. Ce volume convient à la culture de 4 à 5 embryons. Pour un nombre d'embryons supérieur à 5. Le volume de milieu utilisé peut être augmenté à 5 cm3, les dimensions externes sont alors de 4,5 cm de long sur 1,7 cm de diamètre externe, l'épaisseur des parois restant inchangée. De même d'autres formes, autre que la forme cylindrique, peuvent être envisagées: piriforme, ronde ou arciforme en fonction de la plastique du vagin.

Sur la paroi externe du corps du tube, une surface d'inscription pour le nom de la patiente doit être prévue. Sur la paroi externe du bouchon ce qui limite les fautes d'aseptie.

En raison de la diminution d'épaisseur de la paroi du col, le bord externe du bouchon, une fois vissé, ne déborde pas de la paroi du tube.

L'extrémité borgne du tube est arrondie (5).

L'extrémité supérieure en rapport avec le col est plate (6) et présente en son centre un orifice rond de 4 mm de diamètre environ (7). C'est par cet orifice que seront déposés les ovocytes et les spermatozoïdes nécessaires à la fécondation. Les dimensions de l'orifice sont volontairement réduites par rapport au diamètre du tube afin de limiter les échanges du milieu de culture avec l'air ambiant et donc d'en minimiser les perturbations.

Pour diminuer encore les risques septiques, les perturbations du milieu et réduire les temps de manipulations, il nous est apparu bon de concevoir des conteneurs dans lesquels le milieu de culture est placé dans le tube dès sa fabrication (8).

Pour éviter que des perturbations biochimiques du milieu ou qu'une contamination ne surviennent durant le délai séparant sa fabrication et son utilisation, une fermeture hermétique du tube sera réalisée par une fine membrane (9) venant couvrir l'orifice. Cette membrane sera réalisée de telle sorte qu'elle puisse se rompre très facilement lors de la dépose des ovocytes et spermatozoïdes dans le tube à l'aide d'une "pipette Pasteur" en verre ou d'un embout de pipette en plastique à usage unique. D'autres modes de fermeture étanche peuvent être utilisés tel un système muni d'une valve.

Le milieu de culture peut être un de ceux classiquement utilisé et commercialisé. Celui que nous utilisons est le milieu "I.N.R.A. de Menezo" commercialisé sous le nom de "B2" par "A.P.I. Système". Il ne persistera qu'un faible volume gazeux d'environ 200 µl (10) entre la surface du milieu et la membrane, correspondant au volume des ovocytes et spermatozoïdes transférés.

Les parois internes du tube doivent être parfaitement lisses et arrondies (11) afin d'éviter qu'un des oeufs ne reste accroché lorsqu'on les prélèvera pour les replacer dans la cavité utérine.

Le bouchon (12) d'environ 1 cm de haut sur 1,5 cm de diamètre viendra se visser sur le col du tube pour en fermer l'orifice. Après rupture de la membrane une parfaite étanchéité du tube est cependant nécessaire afin d'éviter les perturbations du milieu et les éventuelles contaminations résultant de sa mise en place dans la cavité vaginale. Cette étanchéité sera assurée par 2 joints, l'un en couronne enserrant le col du tube (13) d'une épaisseur d'environ 1 mm; l'autre plat et rond (14) venant tapisser le fond du bouchon et d'une épaisseur identique. Pour en parfaire l'étanchéité le fond du bouchon présente en son milieu un relief (16) dont le diamètre est sensiblement celui de l'orifice et qui permet, lors du vissage du bouchon, de bien appliquer le joint dans la lumière de l'orifice du tube.

La paroi externe du bouchon présente des bords arrondis (15).

Le matériau utilisé pour la réalisation ser aun plastique rigide présentant une grande résistance mécanique (en cas de traumatisme) de type polypropylène non toxique pour les cultures cellulaires.

Le tube et le milieu stériles seront emballés de façon individuelle sous emballage stérile, style papier cellophane; lors de l'emballage le bouchon ne sera pas totalement vissé afin d'éviter une rupture de la membrane. Un dispositif de contention permettant de maintenir le tube dans la cavité vaginale est représenté par la figure 11. Ce dispositif se compose d'un anneau flexible, lui-même constitué d'une lame métallique (17) gainée de caoutchouc (18) à laquelle se trouve attachée une petite poche de caoutchouc (19) de la taille du tube et qui permet de l'y glisser. Le diamètre de cet anneau sera déterminé, comme pour un diaphragme, pour chaque patiente en fonction de la taille de sa cavité utérine et de son col utérin lors d'une consultation avant fécondation in vitro. Ce dispositif permet de maintenir le tube dans le cul de sac vaginal postérieur sans risque pour la patiente de le perdre ou de créer des refroidissements du milieu de culture en rapport avec une position trop proche de l'orifice vulvaire.

La démarche qui nous a permis d'aboutir à ce procédé technique et à son dispositif est basée sur des constatations et travaux de recherche. La première de ces constatations observée pendant 6 mois est qu'une fraction du milieu conservée dans un tube stérile hermétiquement fermé, sans interposition d'air dans la limite du possible, permet de la conserver plus de 10 jours sans perturbation et sans que son utilisation ne modifie les résultats classiquement obtenus. La deuxième constatation repose sur des études de fécondation de 3 ovocytes placés dans le même plot de boite, soit dans 1 ml de milieu; elle montre l'obtention d'un pourcentage de clivage identique à celui obtenu classiquement lorsqu'un seul ovocyte est cultivé par plot. Le fait aussi que si un oeuf est laissé dans le même plot durant les 48 heures nécessaires à sa fécondation, ceci n'interférerait en rien sur son stade de développement.

Enfin des études multiples dont celles effectuées par "Menezo" ont montré des modifications très minimes du milieu de culture avant et après fécondation.

L'ensemble de ces constatations nous ont conduit à pratiquer des études préliminaires qui, bien que le nombre de patientes, le nombre des ovocytes et des oeufs concernés soient faibles nous ont incité à progresser, les résultats initiaux étant très encourageants.

Une première étude portant sur 8 patientes qui présentaient un nombre d'ovocytes supérieur à 4 par ponction a été réalisée. Environ la moitié des oeufs obtenus ont été cultivés de façon classique, l'autre moitié cultivée en tube hermétiquement fermé durant 48 heures à l'étuve. Aucune différence dans le taux de clivage n'a été observée. Deux patientes ont débuté une grossesse, ce qui correspond au pourcentage de succès classiquement obtenu.

Une deuxième série de 8 patientes présentant toujours plus de 4 ovocytes à la ponction, dont environ la moitié ont été cultivé de façon classique, l'autre moitié par la mise en place intra-utérin d'un tube contenant les oeufs. Là encore, les pourcentage de clivage étaient sensiblement équivalents et même légèrement supérieurs pour la technique de culture en intra-vaginale, 5 de ces patientes ont débuté une grossesse.

Bien que ces groupes soient trop petits pour pouvoir les comparer statistiquement et donc qu'il soit trop tôt pour en tirer des conclusions, ces résultats se montrent très encourageants et tout en continuant une étude comparative du mode de culture classique et de celui s'effectuant dans la cavité vaginale, nous avons débuté des cultures intra-vaginales pures avec deux premières tentatives, l'une d'entre elle s'est soldée par une grossesse débutante.

Les intérêts de cette technique sont multiples et considérables: économie de temps et de manipulations diminuant ainsi les risques septiques et la toxicité vis à vis des oeufs. Economie financière importante en rapport avec l'utilisation d'une moins grande quantité de milieu et de petit matériel de laboratoire. Et surtout ne nécessitant plus une étuve à CO2 ou une couveuse, matériels lourds et chers. Simplicité d'utilisation permettant la formation très rapide d'une laborantine expérimentée et permettant ainsi une plus grande diffusion de la technique. Apport psychologique fondamental, la patiente se sentant plus directement concernée par la fécondation de ses oeufs. Il est peut-être trop tôt pour l'envisager, mais les bons résultats d'emblée obtenus sont peut-être en rapport avec une culture s'effectuant à une température qui varie dans le nycthémère et qu'aucune étuve ne peut reproduire actuellement. Cette variation thermique pouvant jouer de façon tout à fait hypothétique un rôle important dans le développement de l'oeuf. Ce procédé permet aussi le transport des ovocytes et des embryons par la patiente elle-même ou dans un réceptacle spécialement conçu pour une température stabilisée à 37°.

## Revendications

1. Procédé de fécondation d'ovocytes in-vitro et de culture des embryons humains, caractérisé en ce qu'il comporte les étapes suivantes en absence d'air enrichi en $CO_2$:

a) on remplit entièrement un conteneur, par un milieu de culture liquide pour ovocytes et embryons et par au moins un ovocyte et des spermatozoïdes; et

b) on ferme hermétiquement le conteneur;

c) on le soumet ainsi fermé à des conditions déterminées de durée et de température afin d'obtenir une fécondation et le clivage du ou des ovocytes fécondés en embryon(s) apte(s) à être implanté(s) dans l'utérus.

2. Procédé suivant la revendication 1, caractérisé en ce qu'à l'étape a) on introduit tout d'abord le volume déterminé du milieu de culture dans le conteneur puis on ferme hermétiquement ce dernier par un moyen de fermeture étanche apte à être ouvert ultérieurement pour l'introduction des gamètes.

3. Procédé suivant la revendication 1 ou 2, caractérisé en ce que à l'étape c) ladite durée est comprise entre 44 et 48 h.

4. Procédé suivant l'une quelconque des revendications 1 à 3, caractérisé en ce que à l'étape c) les conditions déterminées de température sont obtenues en plaçant le conteneur dans un réceptacle dont la température intérieure est d'environ 37°C.

5. Procédé suivant la revendication 4, caractérisé en ce que le réceptacle est un vagin.

6. Procédé suivant la revendication 5, caractérisé en ce qu'on place le conteneur dans le cul de sac postérieur du vagin.

7. Procédé suivant la revendication 6, caractérisé en ce qu'en outre on maintient le conteneur dans cette position à l'aide d'un dispositif de contention.

8. Dispositif de mise en oeuvre du procédé suivant la revendication 5, le dispositif comportant un conteneur ayant une dimension adaptée à son logement dans un vagin, et comprenant un tube, aux parois lisses et arrondies (1, 11), borgne à une extrémité, dite première, l'autre extrémité, dite seconde, comprenant un orifice (7) ainsi que des moyens pour recevoir un bouchon d'étanchéité (12), le tube ayant une cavité interne contenant un milieu de culture liquide pour ovocytes et embryons humains, et adaptée pour recevoir également au moins un ovocyte et des spermatozoïdes, de sorte que, lorsque ladite cavité est remplie avec le milieu de culture et les gamètes, le bouchon (12) assure la fermeture hermétique du conteneur.

9. Dispositif selon la revendication 8, caractérisé en ce que un moyen de fermeture étanche, notamment membrane (9) ou système muni d'une valve, assure la fermeture hermétique du tube dont la cavité contient ledit milieu de culture liquide, ledit moyen de fermeture étant apté à être ouvert pour l'introduction des gamètes dans ladite cavité.

10. Dispositif selon la revendication 9, caractérisé en ce qu'un faible volume gazeux (10) d'environ 200 µl sépare la surface du milieu de culture liquide et ledit moyen de fermeture étanche.

11. Dispositif selon l'une quelconque des revendications 8 à 10, caractérisé en ce que ledit tube a une longueur d'environ 4 à 4,5 cm pour un diamètre externe de 1,5 à 1,7 cm.

12. Dispositif selon l'une quelconque des revendications 8 à 11, caractérisé en ce que la cavité a un volume adapté au nombre d'ovocytes, par exemple environ 3,2 $cm^3$ pour 4 à 5 ovocytes.

13. Dispositif selon l'une quelconque des revendications 8 à 12, caractérisé en ce que ledit orifice (7) est rond et centré par rapport à la seconde extrémité qui se termine par une surface plane (6).

14. Dispositif selon l'une quelconque des revendications 8 à 13, caractérisé en ce que ledit orifice (7) possède un diamètre réduit par rapport au plus grand diamètre interne du tube afin d'éviter le plus possible les perturbations bio-chimiques lors de l'introduction des gamètes dans le tube.

15. Dispositif selon l'une quelconque des revendications 8 à 14, caractérisé en ce que lesdits moyens pour recevoir ledit bouchon d'étanchéité (12) comporte un col (3) muni sur sa paroi externe d'un pas de vis (4) adapté à coopérer avec un pas de vis correspondant aménagé à l'intérieur dudit bouchon (12).

16. Dispositif selon la revendication 15, caractérisé en ce que lesdits moyens pour recevoir ledit bouchon d'étanchéité (12) comportent en outre un joint d'étanchéité en couronne (13) enserrant le col (3) du tube et que ledit bouchon d'étanchéité (12) comporte tapissant son fond un joint d'étanchéité plat et rond (14).

17. Dispositif selon l'une quelconque des revendications 8 à 16, caractérisé en ce que ledit bouchon d'étanchéite (12) comporte des parois externes lisses et arrondies.

18. Dispositif selon la revendication 16 ou 17, caractérisé en ce que ledit fond présente en son milieu un relief (15) d'un diamètre sensiblement identique à celui dudit orifice (7).

19. Dispositif selon l'une quelconque des revendications 8 à 18, caractérisé en ce que ledit bouchon (12), lorsqu'il est en position de fermeture sur le tube, a un bord externe qui ne déborde pas de la paroi externe (1), du tube, le diamètre externe du bouchon (12) étant sensiblement égal au diamètre externe du tube.

20. Dispositif selon l'une quelconque des revendications 8 à 19, caractérisé en ce qu'il comporte en outre un emballage stérile individuel, type poche de cellophane, dudit tube et dudit bouchon d'étanchéité.

21. Dispositif selon l'une quelconque des revendications 8 à 20, caractérisé en ce qu'il comporte de plus un moyen de contention adapté pour le maintient dudit conteneur dans le cul de sac postérieur du vagin.

22. Dispositif selon la revendication 21, caractérisé en ce que le moyen de contention comporte un anneau flexible comprenant une lame métallique (17) gainée de caoutchouc (18) et auquel est fixée une poche de caoutchouc (19) aux dimensions adaptées pour recevoir le conteneur.

## Patentansprüche

1. Verfahren zur in-vitro-Befruchtung menschlicher Eizellen und zur Kultur menschlicher Embryonen, gekennzeichnet durch folgende Verfahrensschritte, die in Abwesenheit von an $CO_2$ angereicherter Luft durchgeführt werden:
a) man füllt einen Container vollständig mit einem flüssigen Kulturmedium für Eizellen und Embryonen und mit mindestens einer Eizelle und Spermatozoen und

b) man verschließt den Container hermetisch und;

c) man unterwirft den so verschlossenen Container Bedingungen, die hinsichtlich Dauer und Temperatur bestimmt sind, um eine Befruchtung und die Tielung der befruchteten Eizelle oder Eizellen zu einem Embryo (Embryonen) zu erhalten, das (die) in den Uterus impantierbar ist (sind).

2. Verfahren nach Anspruch 1, dadurch gekennzeichnet, daß man in der Stufe a) zunächst das bestimmte Volumen des Kulturmediums in den Container einfüllt und diesen dann mittels einer dichten Verschlußvorrichtung hermetisch verschließt, die später zur Einführung von Gameten geöffnet werden kann.

3. Verfahren nach Anspruch 1 oder 2, dadurch gekennzeichnet, daß im Verfahrensschritt c) die Dauer zwischen 44 und 48 Stunden liegt.

4. Verfahren nach einem der Ansprüche 1 bis 3, dadurch gekennzeichnet, daß im Verfahrensschritt c) die bestimmten Bedingungen der Temperatur erhalten werden, indem man den Container in einen Aufnahmebehälter bringt, dessen Innentemperatur etwa 37°C beträgt.

5. Verfahren nach Anspruch 4, dadurch gekennzeichnet, daß der Aufnahmebehälter eine Vagina ist.

6. Verfahren nach Anspruch 5, dadurch gekennzeichnet, daß man den Container in das hintere Gewölbe der Vagina einbringt.

7. Verfahren nach Anspruch 6, dadurch gekennzeichnet, daß man den Container in dieser Lage mit Hilfe einer Haltevorrichtung hält.

8. Vorrichtung zur Durchführung des Verfahrens nach Anspruch 5, wobei die Vorrichtung einen Container mit einer für sein Einsetzen in eine Vagina geeigneten Abmessung aufweist, der ein an einem, dem sogenannten ersten Ende verschlossenes Rohr mit glatten und abgerundeten Wänden (1, 11) aufweist, das am anderen, dem sogenannten zweiten Ende eine Öffnung (7) sowie eine Vorrichtung zur Aufnahme eines dichten Verschlußelements (12) aufweist, wobei das Rohr einen inneren Hohlraum hat, der ein flüssiges Kulturmedium für menschliche Eizellen und Embryonen enthält und auch zur Aufnahme von wenigstens einer Eizelle und Spermatozoen so eingerichtet ist, daß, wenn der Hohlraum mit dem Kulturmedium und den Gameten gefüllt ist, das Verschlußelement (12) für den hermetischen Verschluß des Containers sorgt.

9. Vorrichtung nach Anspruch 8, dadurch gekennzeichnet, daß eine dichte Verschlußvorrichtung, besonders eine Membran (9) oder ein mit einem Ventil versehenes System, die hermetische Abdichtung des Rohrs gewährleistet, dessen Hohlraum das flüssige Kulturmedium enthält, wobei die Vorschlußvorrichtung so ausgebildet ist, daß sie zur Einführung von Gameten in den Hohlraum geöffnet werden kann.

10. Vorrichtung nach Anspruch 9, dadurch gekennzeichnet, daß ein geringes Gasvolumen

(10) von etwa 200 µl eine Oberfläche des flüssigen Kulturmediums von der dichten Verschlußvorrichtung trennt.

11. Vorrichtung nach einem der Ansprüche 8 bis 10, dadurch gekennzeichnet, daß das Rohr eine Länge von etwa 4 bis 4,5 cm bei einem Außendurchmesser von 1,5 bis 1,7 cm hat.

12. Vorrichtung nach einem der Ansprüche 8 bis 11, dadurch gekennzeichnet, daß der Hohlraum ein an die Anzahl von Eizellen angepaßtes Volumen von beispielsweise etwa 3,2 cm³ für 4 bis 5 Eizellen hat.

13. Vorrichtung nach einem der Ansprüche 8 bis 12, dadurch gekennzeichnet, daß die Öffnung (7) rund und bezüglich des zweiten Endes zentriert ist, das in einer ebenen Fläche (6) endet.

14. Verfahren nach einem der Ansprüche 8 bis 13, dadurch gekennzeichnet, daß die Öffnung (7) einen bezüglich des größten Innendurchmessers des Rohrs verringerten Durchmesser aufweist, um soweit wie möglich biochemische Störungen bei der Einführung von Gameten in das Rohr zu verhindern.

15. Vorrichtung nach einem der Ansprüche 8 bis 14, dadurch gekennzeichnet, daß die Vorrichtung zur Aufnahme des dichten Verschlußelements (12) einen Hals (3) aufweist, der an seiner Außenwand mit einem Schraubgewinde (4) versehen ist, das mit einem im Inneren des deckelförmigen Verschlußelements (12) ausgebildeten entsprechenden Gewinde zusammenwirkt.

16. Vorrichtung nach Anspruch 15, dadurch gekennzeichnet, daß die Vorrichtung zur Aufnahme des deckelförmigen dichten Verschlußelements (12) außerdem einen den Hals (3) des Rohrs kranzförmig umgebenden Dichtungsring (13) aufweist und daß das deckelförmige dichte Verschlußelement (12) anliegend an seinem Boden eine ebene runde Dichtungsscheibe (14) aufweist.

17. Vorrichtung nach einem der Ansprüche 8 bis 16, dadurch gekennzeichnet, daß das dichte Verschlußelement (12) glatte und abgerundete Außenwände aufweist.

18. Vorrichtung nach Anspruch 16 oder 17, dadurch gekennzeichnet, daß der Boden in seiner Mitte einen Vorsprung (15) von einem Durchmesser im wesentlichen gleich dem der Öffnung (7) aufweist.

19. Vorrichtung nach einem der Ansprüche 8 bis 18, dadurch gekennzeichnet, daß das dichte Verschlußelement (12), wenn es sich in der Schließstellung auf dem Rohr befindet, einen Außenrand hat, der nicht über die Außenwand (1) des Rohrs vorsteht, und daß der Außendurchmesser des Verschlußelements (12) im wesentlichen gleich dem Außendurchmesser des Rohrs ist.

20. Vorrichtung nach einem der Ansprüche 8 bis 19, dadurch gekennzeichnet, daß sie außerdem eine sterile Einzelverpackung vom Typ Cellophantasche des Rohrs und des dichten Verschlußelements aufweist.

21. Vorrichtung nach einem der Ansprüche 8 bis 20, dadurch gekennzeichnet, daß sie außerdem eine Haltevorrichtung aufweist, die geeignet ist, den Container im hinteren Gewölbe der Vagina zurückzuhalten.

22. Vorrichtung nach Anspruch 21, dadurch gekennzeichnet, daß die Haltevorrichtung einen biegsamen Ring aufweist, der ein mit einer Kautschukumhüllung (18) versehenes Metallblatt (17) aufweist und an dem eine Kautschuktasche (19) mit geeigneten Abmessungen zur Aufnahme des Containers befestigt ist.

**Claims**

1. A process for the fertilisation of ovocytes in vitro and the culturing of human embryos characterised in that it comprises the following steps in the absence of $CO_2$-enriched air:

a) entirely filling a container with a liquid culture medium for ovocytes and embryos and at least one ovocyte and spermatozoa; and

b) hermetically closing the container;

c) when thus closed subjecting it to given conditions in respect of duration and temperature in order to obtain fertilisation and cleavage of the ovocyte or ovocytes which have been fertilised to provide an embryo or embryos suitable for implantation in the uterus.

2. A process according to claim 1 characterised in that step a) involves firstly introducing the given volume of the culture medium into the container and then hermetically closing the latter by a sealing closure means capable of being subsequently opened for the introduction of gametes.

3. A process according to claim 1 or claim 2 characterised in that in step c) said duration is between 44 and 48 hours.

4. A process according to any one of claims 1 to 3 characterised in that in step c) the given temperature conditions are obtained by placing the container in a receptacle, the internal temperature of which is about 37°C.

5. A process according to claim 4 characterised in that the receptacle is a vagina.

6. A process according to claim 5 characterised in that the container is placed in the posterior fornix of the vagina.

7. A process according to claim 6 characterised in that the container is also held in that position by means of a retention device.

8. Apparatus for carrying out the process according to claim 5, the apparatus comprising a container of a size adapted to its being accommodated in a vagina and comprising a tube with smooth rounded walls (1, 11) which is closed at one end, referred to as the first end, the other end, referred to as the second end, comprising an orifice (7) and means for receiving a sealing plug (12), the tube having an internal cavity containing a liquid culture medium for ovocytes and human embryos, and adapted also to receive at least one ovocyte and spermatozoa, in such a way that, when said cavity is filled with the culture medium and gametes, the plug (12) provides for hermetic closure of the container.

9. Apparatus according to claim 8 characterised in that a sealing closure means, in particular a membrane (9) or a system provided with a valve provides for hermetic closure of the tube, the cavity of which contains said liquid culture medium, said closure means being capable of being opened for the introduction of gametes into said cavity.

10. Apparatus according to claim 9 characterised in that a small gaseous volume (10) of about 200 µl separates the surfaces of the liquid culture medium and said sealing closure means.

11. Apparatus according to any one of claims 8 to 10 characterised in that said tube is about 4 to 4.5 cm in length for an outside diameter of 1.5 to 1.7 cm.

12. Apparatus according to any one of claims 8 to 11 characterised in that the cavity is of a volume adapted to the number of ovocytes, for example about 3.2 cm$^3$ for 4 to 5 ovocytes.

13. Apparatus according to any one of claims 8 to 12 characterised in that said orifice (7) is round and centered with respect to the second end which terminates with a flat surface (6).

14. Apparatus according to any one of claims 8 to 13 characterised in that said orifice (7) is of a diameter which is reduced with respect to the largest inside diameter of the tube in order as much as possible to avoid biochemical disturbances upon introduction of the gametes into the tube.

15. Apparatus according to any one of claims 8 to 14 characterised in that said means for receiving said sealing plug (12) comprises a neck (3) provided on its outside wall with a screwthread (4) adapted to co-operate with a corresponding screwthread provided in the interior of said plug (12).

16. Apparatus according to claim 15 characterised in that said means for receiving said sealing plug (12) further comprise a ring sealing joint (13) which surrounds the neck (3) of the tube and that said sealing plug (12) comprises a flat round sealing joint (14) over the end portion thereof.

17. Apparatus according to any one of claims 8 to 16 characterised in that said sealing plug (12) comprises smooth rounded outside walls.

18. Apparatus according to claim 16 or claim 17 characterised in that said end portion has at its centre a raised portion (16) of a diameter which is substantially identical to that of said orifice (7).

19. Apparatus according to any one of claims 8 to 18 characterised in that, when said plug (12) is in the closure position on the tube, it has an outside edge which does not project beyond the outside wall (1) of the tube, the outside diameter of the plug (12) being substantially equal to the outside diameter of the tube.

20. Apparatus according to any one of claims 8 to 19 characterised in that it further comprises a sterile individual package of the cellophane sachet type, for said tube and said sealing plug.

21. Apparatus according to any one of claims 8 to 20 characterised in that it further comprises a retaining means for holding said container in the posterior fornix of the vagina.

22. Apparatus according to claim 21 characterised in that the retaining means comprises a flexible ring having a metal strip (17) which is sheathed with rubber (18) and to which is fixed a rubber pouch (19) of dimensions which are suited to receiving the container.

FIG.1

FIG. 2